# EUROPEAN PATENT APPLICATION

(11) **EP 1 118 679 A1**
(43) Date of publication of application: **25.07.2001**
(21) Application number: 01300271.2
(22) Date of filing: 12.01.2001
(51) Int. Cl.: C12Q 1/68

(54) **Method for diagnosing polymorphisms in the human PDH E2 gene**

(30) Priority: 18.01.2000 GB 0000992
(71) Applicant: AstraZeneca AB, 151 85 Södertälje (SE)
(72) Inventor: Smith, John Craig, Macclesfield, Cheshire SK10 4TG (GB); Anand, Rakesh, Macclesfield, Cheshire SK10 4TG (GB); Morten, John Edward Norris, Macclesfield, Cheshire SK10 4TG (GB)
(74) Representative: Giles, Allen Frank

(57) **Abstract**

This invention relates to polymorphisms in the human pyruvate dehydrogenase complex E2 (PDH E2 or PDC E2) gene and corresponding novel allelic polypeptides encoded thereby. The invention also relates to methods and materials for analysing allelic variation in the PDH E2 gene, and to the use of PDH E2 polymorphism in the diagnosis and treatment of diseases in which modulation of pyruvate dehydrogenase activity could be of therapeutic benefit, such as diabetes, asthma, obesity, sepsis and peripheral vascular disease. In particular, the invention is based on the discovery of two single nucleotide polymorphisms (SNPs) in the coding region of the human PDH E2 gene.

SEQUENCE LISTING

<110> AstraZeneca AB
      Morten, John E
      Smith, John C
      Anand, Rakesh

<120> Methods

<130> PDH E2

<140>
<141>

<160> 2

<170> PatentIn Ver. 2.1

<210> 1
<211> 2583
<212> DNA
<213> Homo sapiens

<400> 1
cgagtgacct cgcgatctgg cccggctccc gctcgtcgca acagcgtgac tacagggtat 60
ggcggggtcc gggcactgtg cggctggacc cccagttctg gggccacgcc gcggaaccgc 120
ttactgctgc agcttttggg gtcgcccggc cgccgctatt acagtcttcc cccgcatcag 180
aaggttccat tgccttctct ttcccccaca atgcaggcag gcaccatagc ccgttggaaa 240
aaaaaagagg gggacaaaat caatgaaggt gacctaattg cagaggttga aactgataaa 300
gccactgttg gatttgagag cctggaggag tgttatatgg caaagatact tgttgctgaa 360
ggtaccaggg atgttcccat cggagcgatc atctgtatca cagttggcaa gcctgaggat 420
attgaggcct ttaaaaatta tacactggat tcctcagcag cacctacccc acaagcggcc 480
ccagcaccaa cccctgctgc cactgcttcg ccacctacac cttctgctca ggctcctggt 540
agctcatatc cccctcacat gcaggtactt cttcctgccc tctctcccac catgaccatg 600
ggcacagttc agagatggga aaaaaaagtg ggtgagaagc taagtgaagg agacttactg 660
gcagagatag aaactgacaa agccactata ggttttgaag tacaggaaga aggttatctg 720
gcaaaaatcc tggtccctga aggcacaaga gatgtccctc taggaacccc actctgtatc 780
attgtagaaa aagaggcaga tatatcagca tttgctgact ataggccaac cgaagtaaca 840
gatttaaaac cacaagtgcc accacctacc ccacccccgg tggccgctgt tcctccaact 900
ccccagcctt tagctcctac accttcagca ccctgcccag ctactcctgc tggaccaaag 960
ggaagggtgt ttgttagccc tcttgcaaag aagttggcag tagagaaagg gattgatctt 1020
acacaagtaa aagggacagg accagatggt agaatcacca agaaggatat cgactctttt 1080
gtgcctagta aagttgctcc tgctccggca gctgttgtgc ctcccacagg tcctggaatg 1140
gcaccagttc ctacaggtgt cttcacagat atcccaatca gcaacattcg tcgggttatt 1200
gcacagcgat taatgcaatc aaagcaaacc atacctcatt attacctttc tatcgatgta 1260
aatatgggag aagttttgtt ggtacggaaa gaacttaata agatattaga agggagaagc 1320
aaaatttctg tcaatgactt catcataaaa gcttcagctt tggcatgttt aaaagttccc 1380
gaagcaaatt cttcttggat ggacacagtt ataagacaaa atcatgttgt tgatgtcagt 1440
gttgcggtca gtactcctgc aggactcatc acacctattg tgtttaatgc acatataaaa 1500
ggagtggaaa ccattgctaa tgatgttgtt tctttagcaa ccaaagcaag agagggtaaa 1560
ctacagccac atgaattcca gggtggcact tttacgatct ccaatttagg aatgtttgga 1620
attaagaatt tctctgctat tattaaccca cctcaagcat gtattttggc aattggtgct 1680
tcagaggata aactggtccc tgcagataat gaaaaagggt ttgatgtggc tagcatgatg 1740
tctgttacac tcagttgtga tcaccgggtg gtggatggag cagttggagc ccagtggctt 1800
gctgagttta gaaagtacct tgaaaaacct atcactatgt tgttgtaact aactcaagaa 1860
tttctaaact ctcccaggtc acactgattc attcttaaca agatatttat atgttattaa 1920
acaggtggtt gctttttatt ttaaccagtt atttttatta ttgagtctgc tcagataagt 1980
tatttataat gggcattact gaatttttaa aatgccgatt acacccaaat attgtgcaca 2040
tttaataatc agacaccaga tttttagctc tgtactccta attaagggac atgtatgtgg 2100
ccttgcctag ccctttggtg ataagtactt cctctaggaa atgtacgata ggtagaattg 2160
tggttcccta aagacaagta cataaaggtg accctgatga aaccttgaag ttctgaaatt 2220
taactgccta aaatgttctc cttagatgtg agagaaagag aaatcagaaa aattaattct 2280
cttgggggaa gggcttgaat tgaagcttta ctttagaatt tagccctggt ttgaaatttt 2340

ccattacatg atcttggttt atcatcgatg ggaagggtag aaaacttcaa ggaaaataag 2400
tgaaatttta aaagtcagca ttttcttaga cctcttcagc tgattgttta tttttctatg 2460
aattctgtac tcaaaattac acatttgttt aaataaatat ccacacaaat tctcagttac 2520
atcaagtagc tggtttatat ttagattatc tcaagtaggg gggaataacc atgtgtagga 2580
att 2583

<210> 2
<211> 545
<212> PRT
<213> Homo sapiens

<400> 2
Met Gln Ala Gly Thr Ile Ala Arg Trp Lys Lys Lys Glu Gly Asp Lys
1               5                   10                  15

Ile Asn Glu Gly Asp Leu Ile Ala Glu Val Glu Thr Asp Lys Ala Thr
            20                  25                  30

Val Gly Phe Glu Ser Leu Glu Glu Cys Tyr Met Ala Lys Ile Leu Val
        35                  40                  45

Ala Glu Gly Thr Arg Asp Val Pro Ile Gly Ala Ile Ile Cys Ile Thr
    50                  55                  60

Val Gly Lys Pro Glu Asp Ile Glu Ala Phe Lys Asn Tyr Thr Leu Asp
65                  70                  75                  80

Ser Ser Ala Ala Pro Thr Pro Gln Ala Ala Pro Ala Pro Thr Pro Ala
                85                  90                  95

Ala Thr Ala Ser Pro Pro Thr Pro Ser Ala Gln Ala Pro Gly Ser Ser
            100                 105                 110

Tyr Pro Pro His Met Gln Val Leu Leu Pro Ala Leu Ser Pro Thr Met
        115                 120                 125

Thr Met Gly Thr Val Gln Arg Trp Glu Lys Lys Val Gly Glu Lys Leu
    130                 135                 140

Ser Glu Gly Asp Leu Leu Ala Glu Ile Glu Thr Asp Lys Ala Thr Ile
145                 150                 155                 160

Gly Phe Glu Val Gln Glu Glu Gly Tyr Leu Ala Lys Ile Leu Val Pro
                165                 170                 175

Glu Gly Thr Arg Asp Val Pro Leu Gly Thr Pro Leu Cys Ile Ile Val
            180                 185                 190

Glu Lys Glu Ala Asp Ile Ser Ala Phe Ala Asp Tyr Arg Pro Thr Glu
        195                 200                 205

Val Thr Asp Leu Lys Pro Gln Val Pro Pro Pro Thr Pro Pro Pro Val
    210                 215                 220

Ala Ala Val Pro Pro Thr Pro Gln Pro Leu Ala Pro Thr Pro Ser Ala
225                 230                 235                 240

Pro Cys Pro Ala Thr Pro Ala Gly Pro Lys Gly Arg Val Phe Val Ser
                245                 250                 255

Pro Leu Ala Lys Lys Leu Ala Val Glu Lys Gly Ile Asp Leu Thr Gln
            260                 265                 270

Val Lys Gly Thr Gly Pro Asp Gly Arg Ile Thr Lys Lys Asp Ile Asp
        275                 280                 285

Ser Phe Val Pro Ser Lys Val Ala Pro Ala Pro Ala Ala Val Val Pro
    290                 295                 300

Pro Thr Gly Pro Gly Met Ala Pro Val Pro Thr Gly Val Phe Thr Asp
305                 310                 315                 320

Ile Pro Ile Ser Asn Ile Arg Arg Val Ile Ala Gln Arg Leu Met Gln
                325                 330                 335

Ser Lys Gln Thr Ile Pro His Tyr Tyr Leu Ser Ile Asp Val Asn Met
            340                 345                 350

Gly Glu Val Leu Leu Val Arg Lys Glu Leu Asn Lys Ile Leu Glu Gly
        355                 360                 365

Arg Ser Lys Ile Ser Val Asn Asp Phe Ile Ile Lys Ala Ser Ala Leu
    370                 375                 380

Ala Cys Leu Lys Val Pro Glu Ala Asn Ser Ser Trp Met Asp Thr Val
385                 390                 395                 400

Ile Arg Gln Asn His Val Val Asp Val Ser Val Ala Val Ser Thr Pro
                405                 410                 415

Ala Gly Leu Ile Thr Pro Ile Val Phe Asn Ala His Ile Lys Gly Val
            420                 425                 430

Glu Thr Ile Ala Asn Asp Val Val Ser Leu Ala Thr Lys Ala Arg Glu
        435                 440                 445

Gly Lys Leu Gln Pro His Glu Phe Gln Gly Gly Thr Phe Thr Ile Ser
    450                 455                 460

Asn Leu Gly Met Phe Gly Ile Lys Asn Phe Ser Ala Ile Ile Asn Pro
465                 470                 475                 480

Pro Gln Ala Cys Ile Leu Ala Ile Gly Ala Ser Glu Asp Lys Leu Val
                485                 490                 495

Pro Ala Asp Asn Glu Lys Gly Phe Asp Val Ala Ser Met Met Ser Val
            500                 505                 510

Thr Leu Ser Cys Asp His Arg Val Val Asp Gly Ala Val Gly Ala Gln
        515                 520                 525

Trp Leu Ala Glu Phe Arg Lys Tyr Leu Glu Lys Pro Ile Thr Met Leu
    530                 535                 540

Leu
545

## Description

This invention relates to polymorphisms in the human pyruvate dehydrogenase complex E2 (PDH E2 or PDC E2) gene and corresponding novel allelic polypeptides encoded thereby. The invention also relates to methods and materials for analysing allelic variation in the PDH E2 gene, and to the use of PDH E2 polymorphism in the diagnosis and treatment of diseases in which modulation of pyruvate dehydrogenase activity could be of therapeutic benefit, such as diabetes, asthma, obesity, sepsis and peripheral vascular disease.

The production of energy for biosynthesis of complex molecules and for muscle contraction is mediated by the hydrolysis of high energy phosphate bonds within adenosine triphosphate (ATP). In oxidative metabolism ATP is generated from acetyl coenzyme A (acetyl CoA), which itself is produced by the beta-oxidation of fatty acids, or as a result of the metabolism of glucose via the glycolytic pathway. The key regulatory enzyme which controls the rate of acetyl CoA formation from glucose is pyruvate dehydrogenase (PDH), which catalyses the oxidation of pyruvate to acetyl CoA and carbon dioxide with concomitant reduction of NAD to NADH.

PDH is a multienzyme complex located in the mitochondrial matrix, containing multiple copies of three enzyme components required to complete the conversion of pyruvate to acetyl CoA (Patel and Roche 1990; FASEB J., 4: 3224-3233). E1 (pyruvate decarboxylase, E.C. 1.2.4.1) catalyses the non-reversible removal of carbon dioxide from pyruvate; E2 (dihydrolipoamide acetyltransferase, E.C. 2.3.1.12) catalyses the formation of acetyl CoA; and E3 (dihydrolipoamide dehydrogenase, E.C. 1.8.1.4) reduces NAD to NADH. The E1 enzyme is a heterotetramer composed of two α and two β subunits. Decarboxylation of pyruvate, catalysed by E1 is the rate limiting step in the overall activity of the PDH complex. This step is also the target for a cycle of phosphorylation and dephosphorylation which forms one of the main mechanisms for regulating PDH activity. Two additional enzyme activities are also associated with the PDH complex: a specific kinase (PDK) which is capable of phosphorylating E1α at three serine residues, and a loosely-associated specific phosphatase which reverses the phosphorylation. Phosphorylation of only one of the three serine residues on E1α renders E1 inactive. Removal of the phosphate groups by the specific phosphatase restores activity. Thus, the proportion of PDH in its active (dephosphorylated) state is determined by a balance between the activity of the kinase and phosphatase. The activity of the kinase may be regulated in vivo by the relative concentrations of metabolic substrates such as NAD/NADH, CoA/acetylCoA and ADP/ATP as well as by the availability of pyruvate itself, therefore providing highly regulated, responsive control of PDH activity.

Genetic abnormalities in the PDH complex are the most common cause of primary lactic acidosis in humans. The majority of cases have been linked with a defect in the E1α subunit. Pathologies associated with defects in the PDH complex conform to a broad clinical spectrum ranging from fatal lactic acidosis in the newborn, to a range of chronic neurodegenerative conditions with gross structural abnormalities in the central nervous system. E1α deficiency is an X-linked disorder which manifests different patterns of clinical presentation between males and females. In addition, heterozygous females show a wide variation in clinical severity of the disease, due largely to variations in the pattern of X-inactivation and differential effects of specific gene mutations on the expression, stability and activity of the mutant protein. A number of mutations in the PDH gene which lead to pyruvate dehydrogenase deficiency have been documented (for a review see NIH OMIM database, reference 312170).

In disease states such as both non-insulin dependent (NIDDM) and insulin-dependent diabetes (IDDM), oxidation of lipids is increased with a concomitant reduction in utilisation of glucose, contributing to the hyperglycaemia. The activity of PDH is reduced in both insulin-dependent and non insulin-dependent diabetes. A further consequence of reduced PDH activity is an increase in pyruvate concentration resulting in increased availability of lactate as a substrate for hepatic gluconeogenesis. Diabetes is further exacerbated by impaired insulin secretion, which has been shown to be associated with reduced PDH activity in pancreatic β-cells. It is believed that increasing the activity of PDH may increase the rate of glucose oxidation and hence overall glucose utilisation, in addition to reducing hepatic glucose output.

Oxidation of glucose is capable of yielding more molecules of ATP per mole of oxygen than is oxidation of fatty acids, therefore in conditions where energy demand may exceed energy supply, such as myocardial ischaemia and reperfusion, intermittent claudication, cerebral ischaemia and reperfusion, shifting the balance of substrate utilisation in favour of glucose metabolism may be expected to improve the ability to maintain ATP levels and hence function. Activation of PDH is predicted to have this effect.

An agent which is capable of activating PDH is expected to be of benefit in treating conditions where an excess of circulating lactic acid is manifest such as in certain cases of sepsis.

The agent dichloroacetic acid which increases the activity of PDH after acute administration in animals (Vary et al., 1988; Circ. Shock, 24: 3-18) has been shown to have the predicted effects in reducing glycaemia (Stacpoole et al, 1978 N. Engl. J. Med. 298, 526-530) and as a therapy for myocardial ischaemia (Bersin and Stacpoole 1997; American Heart Journal, 134: 841-855) and lactic acidaemia (Stacpoole et al, 1983 N. Engl. J. Med 309, 390-396).

cDNA sequences encoding PDH E2 have been submitted to public databases under the following accession numbers:Y00978, J03866. We believe that sequence J03866 contains a number of errors, discussed in more detail in Example 2. All positions in the human PDH E2 gene herein refer to the positions in SEQ ID NO: 1 (which is equivalent to EMBL accession number Y00978 at the time of filing this application) unless stated otherwise or apparent from the context. All positions in the human PDH E2 protein herein refer to the positions in SEQ ID NO: 2 unless stated otherwise or apparent from the context.

DNA polymorphisms may lead to variations in amino acid sequence and consequently to altered protein structure and functional activity. Polymorphisms may also affect mRNA synthesis, maturation, transportation and stability. Polymorphisms which do not result in amino acid changes (silent polymorphisms) or which do not alter any known consensus sequences may nevertheless have a biological effect, for example by altering mRNA folding or stability.

Knowledge of polymorphisms may be used to help identify patients most suited to therapy with particular pharmaceutical agents (this is often termed "pharmacogenetics"). Pharmacogenetics can also be used in pharmaceutical research to assist the drug selection process. Polymorphisms may be used in mapping the human genome and to elucidate the genetic component of diseases. The reader is directed to the following references for background details on pharmacogenetics and other uses of polymorphism detection: Linder *et al*. (1997), Clinical Chemistry, 43, 254; Marshall (1997), Nature Biotechnology, 15, 1249; International Patent Application WO 97/40462, Spectra Biomedical; and Schafer *et al*. (1998), Nature Biotechnology, 16, 33.

Clinical trials have shown that patient response to treatment with pharmaceuticals is often heterogeneous. Thus there is a need for improved approaches to pharmaceutical agent design and therapy.

Variations in polypeptide sequence will be referred to as follows: original amino acid (using one or three letter nomenclature), position, new amino acid. For (a hypothetical) example "D25K" or "Asp25Lys" means that at position 25 an aspartic acid (D) has been changed to lysine (K). Multiple variations in one polypeptide will be shown between square brackets with individual variations separated by commas.

The present invention is based on the discovery of two single nucleotide polymorphisms (SNPs) in the coding region of the human PDH E2 gene.

According to one aspect of the present invention there is provided a method for the diagnosis of a polymorphism in PDH E2 in a human, which method comprises determining the sequence of the human at one or more of positions 857 and 1255 in the PDH E2 gene as defined by the positions in SEQ ID NO: 1 or positions 216 or 349 of PDH E2 protein as defined by the position in SEQ ID NO: 2; and determining the status of the human by reference to polymorphism in PDH E2.

Preferred polymorphisms are any one of the following:

| **Position** | **Polymorphism** | **Reference** |
|---|---|---|
| 857 | T/C | SEQ ID NO: 1 |
| 1255 | G/A | SEQ ID NO: 1 |
| 216 | Val-Ala | SEQ ID NO: 2 |
| 349 | Asp-Asn | SEQ ID NO: 2 |

According to one aspect of the present invention there is provided a method for the diagnosis of a polymorphism in a PDH E2 gene in a human, which method comprises determining the sequence of the nucleic acid of the human at one or more of positions 857 and 1255 in the PDH E2 gene as defined by the positions in SEQ ID NO: 1; and determining the status of the human by reference to polymorphism in the PDH E2 gene.

The term human includes both a human having or suspected of having a PDH-mediated disease and an asymptomatic human who may be tested for predisposition or susceptibility to such disease. At each position the human may be homozygous for an allele or the human may be a heterozygote.

The term 'PDH-mediated disease' means any disease in which changing the level of PDH or changing the activity of PDH would be of therapeutic benefit.

The term 'PDH drug' means any drug which changes the level of PDH or changes the activity of PDH. A drug which increases the activity of PDH is preferred.

The term polymorphism includes single nucleotide substitution, nucleotide insertion and nucleotide deletion, which in the case of insertion and deletion includes insertion or deletion of one or more nucleotides at a position of a gene and variable numbers of a repeated DNA sequence.

In one embodiment of the invention preferably the method for diagnosis described herein is one in which the single nucleotide polymorphism at position 857 in the PDH E2 gene as defined by the positions in SEQ ID NO: 1 is presence of T and/or C.

In another embodiment of the invention preferably the method for diagnosis described herein is one in which the single nucleotide polymorphism at position 1255 in the PDH E2 gene as defined by the positions in SEQ ID NO: 1 is presence of G and/or A.

The method for diagnosis is preferably one in which the sequence is determined by a method selected from amplification refractory mutation system and restriction fragment length polymorphism.

In another aspect of the invention we provide a method for the diagnosis of PDH-mediated disease, which method comprises:
i) obtaining sample nucleic acid from an individual,
ii) detecting the presence or absence of a variant nucleotide at one or more of positions 857 and 1255 in the PDH E2 gene as defined by the positions in SEQ ID NO: 1; and
iii) determining the status of the individual by reference to polymorphism in the PDH E2 gene.

Allelic variation at position 857 in the PDH E2 gene as defined by the positions in SEQ ID NO: 1 consists of a single base substitution from T (the published base), preferably to C. Allelic variation at position 1255 in the PDH E2 gene as defined by the positions in SEQ ID NO: 1 consists of a single base substitution from G (the published base), preferably to A. The status of the individual may be determined by reference to allelic variation at any one or both positions optionally in combination with any other polymorphism in the gene that is (or becomes) known.

The test sample of nucleic acid is conveniently present in a sample of blood, sputum, skin, bronchoalveolar lavage fluid, or other body fluid or tissue obtained from an individual. It will be appreciated that the test sample may equally comprise a nucleic acid sequence corresponding to the sequence in the test sample, that is to say that all or a part of the region in the sample nucleic acid may firstly be amplified using any convenient technique e.g. PCR, before analysis of allelic variation.

It will be apparent to the person skilled in the art that there are a large number of analytical procedures which may be used to detect the presence or absence of variant nucleotides at one or more polymorphic positions of the invention. In general, the detection of allelic variation requires a mutation discrimination technique, optionally an amplification reaction and optionally a signal generation system. Table 1 lists a number of mutation detection techniques, some based on PCR. These may be used in combination with a number of signal generation systems, a selection of which is listed in Table 2. Further amplification techniques are listed in Table 3. Many current methods for the detection of allelic variation are reviewed by Nollau *et al*., Clin. Chem. 43, 1114-1120, 1997; and in standard textbooks, for example "Laboratory Protocols for Mutation Detection", Ed. by U. Landegren, Oxford University Press, 1996 and "PCR", 2^{nd} Edition by Newton & Graham, BIOS Scientific Publishers Limited, 1997.

| **Abbreviations:** | |
|---|---|
| ALEX™ | Amplification refractory mutation system linear extension |
| APEX | Arrayed primer extension |
| ARMS™ | Amplification refractory mutation system |
| b-DNA | Branched DNA |
| CMC | Chemical mismatch cleavage |
| bp | base pair |
| COPS | Competitive oligonucleotide priming system |
| DGGE | Denaturing gradient gel electrophoresis |
| FRET | Fluorescence resonance energy transfer |
| IDDM | Insulin-dependent diabetes mellitus |
| LCR | Ligase chain reaction |
| MASDA | Multiple allele specific diagnostic assay |
| NASBA | Nucleic acid sequence based amplification |
| NIDDM | non-insulin dependent diabetes mellitus |
| OLA | Oligonucleotide ligation assay |
| PCR | Polymerase chain reaction |
| PDH | Pyruvate Dehydrogenase |
| PDK | Pyruvate Dehydrogenase Kinase |
| PDK2 | Pyruvate Dehydrogenase Kinase Isoenzyme 2 |
| PTT | Protein truncation test |
| RFLP | Restriction fragment length polymorphism |
| SDA | Strand displacement amplification |
| SNP | Single nucleotide polymorphism |
| SSCP | Single-strand conformation polymorphism analysis |
| SSR | Self sustained replication |
| TGGE | Temperature gradient gel electrophoresis |
| 3'UTR | 3' Untranslated Region |

**Table 1 -**

| Mutation Detection Techniques |
|---|
| **General:** DNA sequencing, Sequencing by hybridisation |
| **Scanning:** PTT^{*}, SSCP, DGGE, TGGE, Cleavase, Heteroduplex analysis, CMC, Enzymatic mismatch cleavage |
| * Note: not useful for detection of promoter polymorphisms. |
| **Hybridisation Based:** |
| Solid phase hybridisation: Dot blots, MASDA, Reverse dot blots, Oligonucleotide arrays (DNA Chips). |
| Solution phase hybridisation: Taqman™ - US-5210015 & US-5487972 (Hoffmann-La Roche), Molecular Beacons - Tyagi *et al* (1996), Nature Biotechnology, 14, 303; WO 95/13399 (Public Health Inst., New York). |
| **Extension Based:** ARMS™, ALEX™ - European Patent No. EP 332435 B1 (Zeneca Limited), COPS - Gibbs *et al* (1989), Nucleic Acids Research, 17, 2347. |
| **Incorporation Based:** Mini-sequencing, APEX |
| **Restriction Enzyme Based**: RFLP, Restriction site generating PCR |
| **Ligation Based:** OLA |
| **Other**: Invader assay |

**Table 2 -**

| Signal Generation or Detection Systems |
|---|
| **Fluorescence:** FRET, Fluorescence quenching, Fluorescence polarisation - United Kingdom Patent No. 2228998 (Zeneca Limited) |
| **Other:** Chemiluminescence, Electrochemiluminescence, Raman, Radioactivity, Colorimetric, Hybridisation protection assay, Mass spectrometry. |

**Table 3 -**

| Further Amplification Methods |
|---|
| SSR, NASBA, LCR, SDA, b-DNA |

**Table 4-**

| Protein variation detection methods |
|---|
| Immunoassay |
| Immunohistology |
| Peptide sequencing |

Preferred mutation detection techniques include ARMS™, ALEX™, COPS, Taqman, Molecular Beacons, RFLP, and restriction site based PCR and FRET techniques. Immunoassay techniques are known in the art e.g. A Practical Guide to ELISA by D M Kemeny, Pergamon Press 1991; Principles and Practice of Immunoassay, 2^{nd} edition, C P Price & D J Newman, 1997, published by Stockton Press in USA & Canada and by Macmillan Reference in the United Kingdom. Histological techniques are described in Theory and Practice of Histological Techniques by J D Bancroft & A Stevens, 4^{th} Edition, Churchill Livingstone,1996. Protein sequencing is described in Laboratory Techniques in Biochemistry and Molecular Biology, Volume 9, Sequencing of Proteins and Peptides, G Allen, 2^{nd} revised edition, Elsevier, 1989.

Particularly preferred methods include ARMS™ and RFLP based methods. ARMS™ is an especially preferred method.

In a further aspect, the diagnostic methods of the invention are used to assess the efficacy of therapeutic compounds in the treatment of PDH-mediated diseases such as diabetes, asthma, obesity, sepsis, and peripheral vascular disease.

Assays, for example reporter-based assays, may be devised to detect whether one or more of the above polymorphisms affect transcription levels and/or message stability.

Individuals who carry particular allelic variants of the PDH E2 gene may therefore exhibit differences in their ability to regulate protein biosynthesis under different physiological conditions and may display altered abilities to react to different diseases. In addition, differences in protein regulation arising as a result of allelic variation may have a direct effect on the response of an individual to drug therapy. The diagnostic methods of the invention may be useful both to predict the clinical response to such agents and to determine therapeutic dose.

In a further aspect, the diagnostic methods of the invention, are used to assess the predisposition of an individual to diseases mediated by PDH. This may be particularly relevant in the development of diabetes, asthma, obesity, sepsis, and peripheral vascular disease and other diseases which are mediated by PDH. The present invention may be used to recognise individuals who are particularly at risk from developing these conditions.

Low frequency polymorphisms may be particularly useful for haplotyping as described below. A haplotype is a set of alleles found at linked polymorphic sites (such as within a gene) on a single (paternal or maternal) chromosome. If recombination within the gene is random, there may be as many as 2ⁿ haplotypes, where 2 is the number of alleles at each polymorphic position and n is the number of polymorphic positions. One approach to identifying mutations or polymorphisms which are correlated with clinical response is to carry out an association study using all the haplotypes that can be identified in the population of interest. The frequency of each haplotype is limited by the frequency of its rarest allele, so that polymorphisms with low frequency alleles are particularly useful as markers of low frequency haplotypes. As particular mutations or polymorphisms associated with certain clinical features, such as adverse or abnormal events, are likely to be of low frequency within the population, low frequency polymorphisms may be particularly useful in identifying these mutations (for examples see: De Stefano V et al., *Ann Hum Genet* (1998) 62:481-90; and. Keightley AM et al., *Blood* (1999) 93:4277-83).

In a further aspect, the diagnostic methods of the invention are used in the development of new drug therapies which selectively target one or more allelic variants of the PDH E2 gene. Identification of a link between a particular allelic variant and predisposition to disease development or response to drug therapy may have a significant impact on the design of new drugs. Drugs may be designed to regulate the biological activity of variants implicated in the disease process whilst minimising effects on other variants.

In a further diagnostic aspect of the invention the presence or absence of variant nucleotides is detected by reference to the loss or gain of, optionally engineered, sites recognised by restriction enzymes.

According to another aspect of the present invention there is provided a nucleic acid comprising any one of the following polymorphisms:
the nucleic acid of SEQ ID NO: 1 with C at position 857 as defined by the positions in SEQ ID NO: 1;
the nucleic acid of SEQ ID NO: 1 with A at position 1255 as defined by the position in SEQ ID NO: 1;
or a complementary strand thereof or an antisense sequence thereto or a fragment thereof of at least 20 bases comprising at least one polymorphism.

Fragments are at least 17 bases, more preferably at least 20 bases, more preferably at least 30 bases.

The scope of the invention does not extend to any nucleic acid as it is found in nature. A nucleic acid of the invention is preferably in isolated form, for example through being at least partially purified from any substance with which it occurs naturally (if any).

Novel sequence disclosed herein, may be used in another embodiment of the invention to regulate expression of the gene in cells by the use of antisense constructs. To enable methods of down-regulating expression of the gene of the present invention in mammalian cells, an example antisense expression construct can be readily constructed for instance using the pREP10 vector (Invitrogen Corporation). Transcripts are expected to inhibit translation of the gene in cells transfected with this type of construct. Antisense transcripts are effective for inhibiting translation of the native gene transcript, and capable of inducing the effects (e.g., regulation of tissue physiology) herein described. Oligonucleotides which are complementary to and hybridisable with any portion of novel gene mRNA disclosed herein are contemplated for therapeutic use. U.S. Patent No. 5,639,595, "Identification of Novel Drugs and Reagents", issued Jun. 17, 1997, wherein methods of identifying oligonucleotide sequences that display in vivo activity are thoroughly described, is herein incorporated by reference. Expression vectors containing random oligonucleotide sequences derived from previously known polynucleotides are transformed into cells. The cells are then assayed for a phenotype resulting from the desired activity of the oligonucleotide. Once cells with the desired phenotype have been identified, the sequence of the oligonucleotide having the desired activity can be identified. Identification may be accomplished by recovering the vector or by polymerase chain reaction (PCR) amplification and sequencing the region containing the inserted nucleic acid material. Antisense molecules can be synthesised for antisense therapy. These antisense molecules may be DNA, stable derivatives of DNA such as phosphorothioates or methylphosphonates, RNA, stable derivatives of RNA such as 2'-O-alkylRNA, or other oligonucleotide mimetics. U.S. Patent No. 5,652,355, "Hybrid Oligonucleotide Phosphorothioates", issued July 29, 1997, and U.S. Patent No. 5,652,356, "Inverted Chimeric and Hybrid Oligonucleotides", issued July 29, 1997, which describe the synthesis and effect of physiologically-stable antisense molecules, are incorporated by reference. Antisense molecules may be introduced into cells by microinjection, liposome encapsulation or by expression from vectors harboring the antisense sequence.

The invention further provides nucleotide primers which can detect the polymorphisms of the invention.

According to another aspect of the present invention there is provided an allele specific primer capable of detecting a PDH E2 gene polymorphism at one or more of positions 857 and 1255 in the PDH E2 gene as defined by the positions in SEQ ID NO: 1.

An allele specific primer is used, generally together with a constant primer, in an amplification reaction such as a PCR reaction, which provides the discrimination between alleles through selective amplification of one allele at a particular sequence position e.g. as used for ARMS™ assays. The allele specific primer is preferably 17- 50 nucleotides, more preferably about 17-35 nucleotides, more preferably about 17-30 nucleotides.

An allele specific primer preferably corresponds exactly with the allele to be detected but derivatives thereof are also contemplated wherein about 6-8 of the nucleotides at the 3' terminus correspond with the allele to be detected and wherein up to 10, such as up to 8, 6, 4, 2, or 1 of the remaining nucleotides may be varied without significantly affecting the properties of the primer.

Primers may be manufactured using any convenient method of synthesis. Examples of such methods may be found in standard textbooks, for example "Protocols for Oligonucleotides and Analogues; Synthesis and Properties," Methods in Molecular Biology Series; Volume 20; Ed. Sudhir Agrawal, Humana ISBN: 0-89603-247-7; 1993; 1^{st} Edition. If required the primer(s) may be labelled to facilitate detection.

According to another aspect of the present invention there is provided an allele-specific oligonucleotide probe capable of detecting a PDH E2 gene polymorphism at one or more of positions 857 and 1255 in the PDH E2 gene as defined by the positions in SEQ ID NO: 1.

The allele-specific oligonucleotide probe is preferably 17- 50 nucleotides, more preferably about 17-35 nucleotides, more preferably about 17-30 nucleotides.

The design of such probes will be apparent to the molecular biologist of ordinary skill. Such probes are of any convenient length such as up to 50 bases, up to 40 bases, more conveniently up to 30 bases in length, such as for example 8-25 or 8-15 bases in length. In general such probes will comprise base sequences entirely complementary to the corresponding wild type or variant locus in the gene. However, if required one or more mismatches may be introduced, provided that the discriminatory power of the oligonucleotide probe is not unduly affected. The probes of the invention may carry one or more labels to facilitate detection.

According to another aspect of the present invention there is provided a diagnostic kit comprising an allele specific oligonucleotide probe of the invention and/or an allele-specific primer of the invention.

The diagnostic kits may comprise appropriate packaging and instructions for use in the methods of the invention. Such kits may further comprise appropriate buffer(s), nucleotides, and polymerase(s) such as thermostable polymerases, for example taq polymerase.

In another aspect of the invention, the polymorphisms of this invention may be used as a genetic markers in a linkage study. This particularly applies to the polymorphism at position 1255 in the PDH E2 gene as defined by the positions in SEQ ID NO: 1 because of its relatively high frequency (see Examples below).

According to another aspect of the present invention there is provided a method of treating a human in need of treatment with a PDH drug in which the method comprises:
i) diagnosis of a polymorphism in PDH E2 in a human, which method comprises determining the sequence of the human at one or more of positions 857 and 1255 in the PDH E2 gene as defined by the positions in SEQ ID NO: 1 or positions 216 or 349 of PDH E2 protein as defined by the position in SEQ ID NO: 2; and determining the status of the human by reference to polymorphism in the PDH E2 gene; and
ii) administering an effective amount of a PDH drug.

According to another aspect of the present invention there is provided a method of treating a human in need of treatment with a PDH drug in which the method comprises:
i) diagnosis of a polymorphism in the PDH E2 gene in the human, which diagnosis comprises determining the sequence of the nucleic acid at one or more of positions 857 and 1255 in the PDH E2 gene as defined by the positions in SEQ ID NO: 1, and determining the status of the human by reference to polymorphism in the PDH E2 gene; and
ii) administering an effective amount of a PDH drug.

Preferably determination of the status of the human is clinically useful. Examples of clinical usefulness include deciding which drug or drugs to administer and/or establishing the effective amount of the drug or drugs.

Drugs which increase the activity of PDH are of value in a number of disease conditions, including disease states associated with disorders of glucose utilisation such as diabetes and obesity, and associated with excessive production of lactate such as encountered in sepsis and other causes of lactic acidaemia. Additionally drugs which increase the activity of PDH may be expected to have utility in diseases where supply of energy-rich substrates to tissues is limiting such as peripheral vascular disease, coronary failure and certain cardiac myopathies, muscle ataxia and weakness.

According to another aspect of the present invention there is provided use of a PDH drug in the preparation of a medicament for treating a PDH-mediated disease in a human diagnosed as having a polymorphism at one or more of positions 857 and 1255 in the PDH E2 gene as defined by the positions in SEQ ID NO: 1.

According to another aspect of the present invention there is provided use of a PDH drug in the preparation of a medicament for treating a PDH-mediated disease in a human diagnosed as having a a polymorphism in PDH E2 in a human, which method comprises determining the sequence of the human at one or more of positions 857 and 1255 in the PDH E2 gene as defined by the positions in SEQ ID NO: 1 or positions 216 or 349 of PDH E2 protein as defined by the position in SEQ ID NO: 2.

According to another aspect of the present invention there is provided a pharmaceutical pack comprising a PDH drug and instructions for administration of the drug to humans diagnostically tested for a polymorphism at one or more of positions 857 and 1255 in the PDH E2 gene as defined by the positions in SEQ ID NO: 1.

According to another aspect of the present invention there is provided a pharmaceutical pack comprising a PDH drug and instructions for administration of the drug to humans diagnostically tested for a polymorphism in PDH E2 in a human, which method comprises determining the sequence of the human at one or more of positions 857 and 1255 in the PDH E2 gene as defined by the positions in SEQ ID NO: 1 or positions 216 or 349 of PDH E2 protein as defined by the position in SEQ ID NO: 2.

According to another aspect of the present invention there is provided a computer readable medium comprising at least one novel polynucleotide sequence of the invention stored on the medium. The computer readable medium may be used, for example, in homology searching, mapping, haplotyping, genotyping or pharmacogenetic analysis or any other bioinformatic analysis. The reader is referred to Bioinformatics, A practical guide to the analysis of genes and proteins, Edited by A D Baxevanis & B F F Ouellette, John Wiley & Sons, 1998. Any computer readable medium may be used, for example, compact disk, tape, floppy disk, hard drive or computer chips.

The polynucleotide sequences of the invention, or parts thereof, particularly those relating to and identifying the polymorphisms identified herein represent a valuable information source, for example, to characterise individuals in terms of haplotype and other sub-groupings, such as investigation of susceptibility to treatment with particular drugs. These approaches are most easily facilitated by storing the sequence information in a computer readable medium and then using the information in standard bioinformatics programs or to search sequence databases using state of the art searching tools such as "GCC". Thus, the polynucleotide sequences of the invention are particularly useful as components in databases useful for sequence identity and other search analyses. As used herein, storage of the sequence information in a computer readable medium and use in sequence databases in relation to 'polynucleotide or polynucleotide sequence of the invention' covers any detectable chemical or physical characteristic of a polynucleotide of the invention that may be reduced to, converted into or stored in a tangible medium, such as a computer disk, preferably in a computer readable form. For example, chromatographic scan data or peak data, photographic scan or peak data, mass spectrographic data, sequence gel (or other) data.

The invention provides a computer readable medium having stored thereon one or more polynucleotide sequences of the invention. For example, a computer readable medium is provided comprising and having stored thereon a member selected from the group consisting of: a polynucleotide comprising the sequence of a polynucleotide of the invention, a polynucleotide consisting of a polynucleotide of the invention, a polynucleotide which comprises part of a polynucleotide of the invention, which part includes at least one of the polymorphisms of the invention, a set of polynucleotide sequences wherein the set includes at least one polynucleotide sequence of the invention, a data set comprising or consisting of a polynucleotide sequence of the invention or a part thereof comprising at least one of the polymorphisms identified herein.

A computer based method is also provided for performing sequence identification, said method comprising the steps of providing a polynucleotide sequence comprising a polymorphism of the invention in a computer readable medium; and comparing said polymorphism containing polynucleotide sequence to at least one other polynucleotide or polypeptide sequence to identify identity (homology), i.e. screen for the presence of a polymorphism.

Another aspect of the invention comprises use of any polymorphism as defined herein in bioinformatic analysis. Preferably the bioinformatic analysis is selected from homology searching, mapping, haplotyping, genotyping or pharmacogenetic analysis.

According to another aspect of the present invention there is provided an allelic variant of the human PDH E2 polypeptide having a alanine at position 216 and/or an asparagine at position 349 or a fragment thereof comprising at least 10 amino acids provided that the fragment comprises the allelic variant at position 216 and/or position 349.

Fragments of PDH E2 polypeptide are at least 10 amino acids, more preferably at least 15 amino acids, more preferably at least 20 amino acids. The polypeptide of the invention does not encompass naturally occuring polypeptide as it occurs in nature, for example, the polypeptide is at least partially purified from at least one component with which it occurs naturally. Preferably the polypeptide is at least 30% pure, more preferably at least 60% pure, more preferably at least 90% pure, more preferably at least 95% pure, and more preferably at least 99% pure.

According to another aspect of the present invention there is provided an antibody specific for an allelic variant of human PDH E2 polypeptide having a alanine at position 216 and/or an asparagine at position 349 or a fragment thereof comprising at least 10 amino acids provided that the fragment comprises the allelic variants at position 216 and /or position 349.

Antibodies can be prepared using any suitable method. For example, purified polypeptide may be utilised to prepare specific antibodies. The term "antibodies" is meant to include polyclonal antibodies, monoclonal antibodies, and the various types of antibody constructs such as for example F(ab')₂, Fab and single chain Fv. Antibodies are defined to be specifically binding if they bind the T679M variant of integrin α₄ with a Kₐ of greater than or equal to about 10⁷ M⁻¹. Affinity of binding can be determined using conventional techniques, for example those described by Scatchard et al., *Ann*. *N*.*Y*. *Acad*. *Sci.,* 51:660 (1949).

Polyclonal antibodies can be readily generated from a variety of sources, for example, horses, cows, goats, sheep, dogs, chickens, rabbits, mice or rats, using procedures that are well-known in the art. In general, antigen is administered to the host animal typically through parenteral injection. The immunogenicity of antigen may be enhanced through the use of an adjuvant, for example, Freund's complete or incomplete adjuvant. Following booster immunisations, small samples of serum are collected and tested for reactivity to antigen. Examples of various assays useful for such determination include those described in: *Antibodies: A Laboratory Manual,* Harlow and Lane (eds.), Cold Spring Harbor Laboratory Press, 1988; as well as procedures such as countercurrent immuno-electrophoresis (CIEP), radioimmunoassay, radioimmunoprecipitation, enzyme-linked immuno-sorbent assays (ELISA), dot blot assays, and sandwich assays, see U.S. Patent Nos. 4,376,110 and 4,486,530.

Monoclonal antibodies may be readily prepared using well-known procedures, see for example, the procedures described in U.S. Patent Nos. RE 32,011, 4,902,614, 4,543,439 and 4,411,993; Monoclonal Antibodies, Hybridomas: *A New Dimension in Biological Analyses,* Plenum Press, Kennett, McKearn, and Bechtol (eds.), (1980).

The monoclonal antibodies of the invention can be produced using alternative techniques, such as those described by Alting-Mees et al., "Monoclonal Antibody Expression Libraries: A Rapid Alternative to Hybridomas", *Strategies in Molecular Biology* 3: 1-9 (1990) which is incorporated herein by reference. Similarly, binding partners can be constructed using recombinant DNA techniques to incorporate the variable regions of a gene that encodes a specific binding antibody. Such a technique is described in Larrick et al., *Biotechnology,* 7: 394 (1989).

Once isolated and purified, the antibodies may be used to detect the presence of antigen in a sample using established assay protocols.

The invention will now be illustrated but not limited by reference to the following Examples. All temperatures are in degrees Celsius.

In the Examples below, unless otherwise stated, the following methodology and materials have been applied.

AMPLITAQ™ or AMPLITAQ GOLD™ available from Perkin-Elmer Cetus, are used as the source of thermostable DNA polymerase.

General molecular biology procedures can be followed from any of the methods described in "Molecular Cloning - A Laboratory Manual" Second Edition, Sambrook, Fritsch and Maniatis (Cold Spring Harbor Laboratory, 1989) or "Current Protocols in Molecular Biology", Volumes1-3 , Edited by F M Asubel, R Brent & R E Kingston, published by John Wiley, 1998.

Electropherograms were obtained in a standard manner: data was collected by ABI377 data collection software and the wave form generated by ABI Prism™ sequencing analysis (2.1.2).

### Example 1

### Identification of Polymorphisms

### 1. Methods

### c-DNA Preparation

RNA was prepared from lymphoblastoid cell lines from Caucasian donors using standard laboratory protocols (Chomczynski and Sacchi, Anal. Biochem. 162, 156-159, 1987) and used to generate first strand cDNA (Gubler and Hoffman, Gene 25, 263-269, 1983).

### Template Preparation

Templates were prepared by PCR using the oligonucleotide primers and annealing temperatures set out below. The extension temperature was 72° and denaturation temperature 94°; each step was 1 minute. Generally 100 pg cDNA was used in each reaction and subjected to 40 cycles of PCR.

| **Fragment** | **Forward Oligo** | **Reverse Oligo** | **Annealing Temp** | **DMSO** | **MgCl**_{**2**} |
|---|---|---|---|---|---|
| 176-579 | 176-198 | 559-579 | 55° | 5% | 1.5mM |
| 455-998 | 455-475 | 975-998 | 60° | 0% | 1.5mM |
| 815-1206 | 815-836 | 1185-1206 | 62° | 0% | 1mM |
| 1109-1592 | 1109-1128 | 1570-1592 | 62° | 0% | 1mM |
| 1429-1893 | 1429-1450 | 1871-1893 | 62° | 0% | 1mM |

All positions refer to the positions in SEQ ID NO: 1.

For dye-primer sequencing the forward primers were modified to include M13 forward sequence (ABI protocol P/N 402114, Applied Biosystems) at the 5' end of the oligonucleotides.

### Dye Primer Sequencing

Dye-primer sequencing using M13 forward primer was as described in the ABI protocol P/N 402114 for the ABI Prism™ dye primer cycle sequencing core kit with "AmpliTaq FS"™ DNA polymerase, modified in that the annealing temperature was 45° and DMSO was added to the cycle sequencing mix to a final concentration of 5%.

The extension reactions for each base were pooled, ethanol/sodium acetate precipitated, washed and resuspended in formamide loading buffer.

4.25% Acrylamide gels were run on an automated sequencer (ABI 377, Applied Biosystems).

### 2. Results

### Novel Polymorphisms

| Position | Published allele | Variant allele | amino acid change | RFLP | Allele Frequency |
|---|---|---|---|---|---|
| 857 | T | C | Val-Ala | + Hha I | 11/40 |
| 1255 | G | A | Asp-Asn | - Cla I | 17/38 |

All positions refer to the positions in SEQ ID NO: 1.
Frequency is the allele frequency of the variant allele in control subjects.

### Summary of polymorphisms:

Position 857 of SEQ ID NO: 1, GTG (Val at 216 of SEQ ID NO: 2) to GCG (Ala at 216 of SEQ ID NO: 2); and

Position 1255 of SEQ ID NO: 1 GAT (Asp at 349 of SEQ ID NO: 2) to AAT (Asn at 349 of SEQ ID NO: 2).

### Example 2

### Analysis of the sequences of PDH E2 disclosed in public databases

Two cDNA sequences encoding PDH E2 have been submitted to public databases under accession numbers Y00978 and J03866. Y00978 identifies an open reading frame (ORF) which extends previously published sequence at the 5' end but does not identify an ATG start site. J03866 contains more 5' sequence than Y00978 and identifies an ATG start site. However, the 5' sequence of J03866 does not match that of Y00978.

We used sequence regions common to Y00978 and J03866 to search public and proprietary sequence databases and were able to obtain extended sequence at the 5' end. The extended sequence identified the start of an open reading frame which was in frame with the ORFs reported within both Y00978 and J03866. The initiating ATG corresponded to position 211 of Y00978, position 843 of J03866.

Sequence at positions 1-698 of J03866 did not match the extended sequence determined in our analysis. Sequence J03866 1-698 was found to be identical to murine sulphatase A. Furthermore, we were unable to amplify the predicted PCR product sizes from human lymphoblastoid cell line cDNA using PCR primers designed from sequence J03866 1-698, providing further confirmation that sequence J03866 1-698 is incorrect. J03866 also differs from Y00978 by three missing nucleotides.

Sequence analysis of lymphoblastoid cell line cDNA agreed with the sequence of Y00978. In conclusion, we believe that sequence Y00978 is correct and that the ATG start site corresponds to position 211 of Y00978.

## Claims

1. A method for the diagnosis of a polymorphism in PDH E2 in a human, which method comprises determining the sequence of the human at one or more of positions 857 and 1255 in the PDH E2 gene as defined by the positions in SEQ ID NO: 1 or positions 216 or 349 of PDH E2 protein as defined by the position in SEQ ID NO: 2; and determining the status of the human by reference to polymorphism in PDH E2.

2. A method according to claim 1 in which the polymorphisms are further defined as:
| **Position** | **Polymorphism** | **Reference** |
|---|---|---|
| 857 | T/C | SEQ ID NO: 1 |
| 1255 | G/A | SEQ ID NO: 1 |
| 216 | Val-Ala | SEQ ID NO: 2 |
| 349 | Asp-Asn | SEQ ID NO: 2 |

3. An isolated nucleic acid comprising any one of the following polymorphisms:
the nucleic acid of SEQ ID NO: 1 with C at position 857 as defined by the positions in SEQ ID NO: 1;
the nucleic acid of SEQ ID NO: 1 with A at position 1255 as defined by the position in SEQ ID NO: 1; or a complementary strand thereof or an antisense sequence thereto or a fragment thereof of at least 20 bases comprising at least one polymorphism.

4. An allele specific primer capable of detecting a PDH E2 gene polymorphism at one or more of positions 857 and 1255 in the PDH E2 gene as defined by the positions in SEQ ID NO: 1.

5. An allele-specific oligonucleotide probe capable of detecting a PDH E2 gene polymorphism at one or more of positions 857 and 1255 in the PDH E2 gene as defined by the positions in SEQ ID NO: 1.

6. A diagnostic kit comprising an allele specific oligonucleotide probe as claimed in claim 5 and/or an allele-specific primer as claimed in claim 4.

7. Use of any polymorphism defined in claim 2 as a genetic marker in a linkage study.

8. Use of a PDH drug in the preparation of a medicament for treating a PDH-mediated disease in a human diagnosed as having a polymorphism in PDH E2 in a human, which method comprises determining the sequence of the human at one or more of positions 857 and 1255 in the PDH E2 gene as defined by the positions in SEQ ID NO: 1 or positions 216 or 349 of PDH E2 protein as defined by the position in SEQ ID NO: 2.

9. An allelic variant of the human PDH E2 polypeptide in isolated form having a alanine at position 216 and/or an asparagine at position 349 or a fragment thereof comprising at least 10 amino acids provided that the fragment comprises the allelic variant at position 216 and/or position 349.

10. An antibody specific for an allelic variant of human PDH E2 polypeptide having a alanine at position 216 and/or an asparagine at position 349 or a fragment thereof comprising at least 10 amino acids provided that the fragment comprises the allelic variant at position 216 and /or position 349.

11. Use of any polymorphism as defined in claim 1 or 2 in bioinformatic analysis.

12. A use according to claim 11 comprising a bioinformatic analysis selected from homology searching, mapping, haplotyping, genotyping or pharmacogenetic analysis.
